(19)

Europäisches
Patentamt
European
Patent Office
Office européen
des brevets

(11) **EP 4 590 203 B1**

(12) **EUROPEAN PATENT SPECIFICATION**

(45) Date of publication and mention
of the grant of the patent:
**17.12.2025  Bulletin 2025/51**

(21) Application number: **23776261.2**

(22) Date of filing: **07.09.2023**

(51) International Patent Classification (IPC):
***A61B 8/08*** *(2006.01)*

(52) Cooperative Patent Classification (CPC):
**A61B 8/0858; A61B 8/0833; A61B 8/5223**

(86) International application number:
**PCT/EP2023/074522**

(87) International publication number:
**WO 2024/061635 (28.03.2024 Gazette 2024/13)**

(54) **INCLINATION ANGLE CORRECTION FOR ULTRASOUND-BASED DIAPHRAGM THICKNESS MEASUREMENTS**

NEIGUNGSWINKELKORREKTUR FÜR ULTRASCHALLGESTÜTZTE MESSUNGEN DER DIAPHRAGMA-DICKE

CORRECTION DE L'ANGLE D'INCLINAISON POUR LES MESURES DE L'ÉPAISSEUR DU DIAPHRAGME PAR ULTRASONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **19.09.2022   US 202263407769 P**

(43) Date of publication of application:
**30.07.2025   Bulletin 2025/31**

(73) Proprietor: **Koninklijke Philips N.V.
5656 AG Eindhoven (NL)**

(72) Inventors:
- **KOEHLER, Thomas
  5656 AG Eindhoven (NL)**
- **WIEMKER, Rafael
  5656 AG Eindhoven (NL)**
- **HENDRIKS, Cornelis Petrus
  5656 AG Eindhoven (NL)**
- **POLKEY, Michael
  5656 AG Eindhoven (NL)**
- **SABCZYNSKI, Joerg
  5656 AG Eindhoven (NL)**
- **BUIZZA, Roberto
  5656 AG Eindhoven (NL)**
- **HAARTSEN, Jaap Roger
  5656 AG Eindhoven (NL)**
- **WIEBERNEIT, Nataly
  5656 AG Eindhoven (NL)**

(74) Representative: **Philips Intellectual Property & Standards
High Tech Campus 52
5656 AG Eindhoven (NL)**

(56) References cited:
**WO-A2-2022/128759     CN-A- 114 680 929**

- **TUINMAN PIETER R ET AL: "Respiratory muscle ultrasonography: methodology, basic and advanced principles and clinical applications in ICU and ED patients-a narrative review", INTENSIVE CARE MEDICINE, SPRINGER BERLIN HEIDELBERG, BERLIN/HEIDELBERG, vol. 46, no. 4, 14 January 2020 (2020-01-14), pages 594 - 605, XP037077546, ISSN: 0342-4642, [retrieved on 20200114], DOI: 10.1007/ S00134-019-05892-8**

**EP 4 590 203 B1**

**(Cont. next page)**

• MATAMIS DIMITRIOS ET AL: "Sonographic evaluation of the diaphragm in critically ill patients. Technique and clinical applications", INTENSIVE CARE MEDICINE, SPRINGER BERLIN HEIDELBERG, BERLIN/HEIDELBERG, vol. 39, no. 5, 24 January 2013 (2013-01-24), pages 801 - 810, XP037119915, ISSN: 0342-4642, [retrieved on 20130124], DOI: 10.1007/S00134-013-2823-1

**Description**

## CROSS-REFERENCE TO RELATED APPLICATIONS

This patent application claims the priority benefit under 35 U.S.C. § 119(e) of U.S. Provisional Application No. 63/407,769, filed on September 19, 2002.

The following relates generally to the respiratory therapy arts, mechanical ventilation arts, ventilator induced lung injury (VILI) arts, ultrasound probe arts, and related arts.

## BACKGROUND

Diaphragmatic ultrasonography (US) allows for quantification of diaphragm thickness, strain (rate) and excursion, and with this also the respiratory rate and duration of each contraction. Diaphragm thickness (expressed as thickening fraction) and strain reflect contractile activity and correlate well with diaphragmatic electrical activity and diaphragmatic pressure. Consequently, thickness and strain may be used as a surrogate for respiratory effort. Applications of diaphragmatic ultrasound include assessment of diaphragm function, atrophy detection, weaning prediction, and mechanical ventilation (MV) setting management. Other applications could be asynchrony detection and proportional ventilation (non-invasive neurally adjusted ventilatory assist (NAVA)). The use of diaphragmatic ultrasound in mechanical ventilation is gaining attention and therefore, technical problems and use cases are currently being investigated.

A diaphragm thickening fraction (TFdi or TFDI) as measured by ultrasound (US) are carried out by an operator who looks at the patient and takes an ultrasound image at end inhalation and end exhalation. The diaphragm thickness fraction is determined by subtracting the end inhalation thickness from the end exhalation thickness and dividing the difference by the exhale thickness according to Equation 1:

$$TFdi = \frac{T_{ei} - T_{ee}}{T_{ee}} * 100\% \qquad (1)$$

with $T_{ei}$ as the end-inspiratory thickness. The thickness of the diaphragm as varying over the breathing cycle (i.e. the thickening during inspiration) is a surrogate for the patient's respiratory effort (see, e.g., Tuinman PR, Jonkman AH, Dres M, Shi ZH, Goligher EC, Goffi A, de Korte C, Demoule A, Heunks L. Respiratory muscle ultrasonography: methodology, basic and advanced principles and clinical applications in ICU and ED patients - a narrative review. Intensive Care Med. 2020 Apr;46(4):594-605. doi: 10.1007/s00134-019-05892-8. Epub 2020 Jan 14. PMID: 31938825; PMCID: PMC7103016.). CN114680929A discloses an ultrasound imaging method and system for measuring diaphragm.

MATAMIS DIMITRIOS ET AL: "Sonographic evaluation of the diaphragm in critically ill patients. Technique and clinical applications", INTENSIVE CARE MEDICINE, SPRINGER BERLIN HEIDELBERG, BERLIN/HEIDELBERG, vol. 39, no. 5, 24 January 2013, pages 801-810,ISSN: 0342-4642, DOI: 10.1007/S00134-013-2823-1 describes the technique and clinical applications of sonographic evaluation of the diaphragm in critically ill patients.

Diaphragm thickness measurements can be obtained with ultrasound (US) imaging. However, such measurements can suffer from artificial thickening if the US beam does not pass the diaphragm perpendicularly. If the US beam has an inclination with respect to the surface normal of $\alpha$, then the diaphragm thickness appears to be $d/\cos\alpha$, i.e., it appears to be thicker by a factor of $1/\cos\alpha$ than it actually is. This can lead to inaccurate diaphragm thickness measurements.

The following discloses certain improvements to overcome these problems and others.

## SUMMARY

In one aspect, a diaphragm imaging device includes at least one electronic processor programmed to perform a diaphragm imaging method including receiving ultrasound imaging data of a diaphragm of a patient, the ultrasound imaging data being acquired by an associated ultrasound imaging probe with the probe at a plurality of different observable probe angles ($\beta_{obs}$); for each observable probe angle, determining a corresponding apparent thickness ($d_l$) of the diaphragm of the patient from the received ultrasound data acquired at that observable probe angle; and estimating a thickness ($d_D$) of the diaphragm of the patient based at least on the apparent thicknesses ($d_l$).

In another aspect, a diaphragm imaging method includes, with at least one electronic controller, receiving ultrasound imaging data of a diaphragm of a patient, the ultrasound imaging data being acquired by an associated ultrasound imaging probe with the probe at a plurality of different observable probe angles ($\beta_{obs}$); for each observable probe angle, determining a corresponding apparent thickness ($d_l$) of the diaphragm of the patient from the received ultrasound data acquired at that observable probe angle; and estimating a thickness ($d_D$) of the diaphragm of the patient based at least on the apparent thicknesses ($d_l$).

One advantage resides in acquiring accurate diaphragm thickness measurements.

Another advantage resides in correcting an angle of an ultrasound probe that is imaging a diaphragm to obtain an accurate diaphragm thickness measurement.

Another advantage resides in providing feedback to a user to correct an inclination angle of an ultrasound probe while imaging a diaphragm.

Another advantage resides in correcting an artificial thickening factor in a diaphragm thickness measurement.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically shows an illustrative diaphragm imaging device in accordance with the present disclosure.

FIGURE 2 shows a different embodiments of a probe of the device of FIGURE 1.

FIGURES 3-5 show example operations of the probe of FIGURE 1.

FIGURE 6 shows an example flow chart of operations suitably performed by the device of FIGURE 1.

## DETAILED DESCRIPTION

As used herein, the singular form of "a," "an," and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

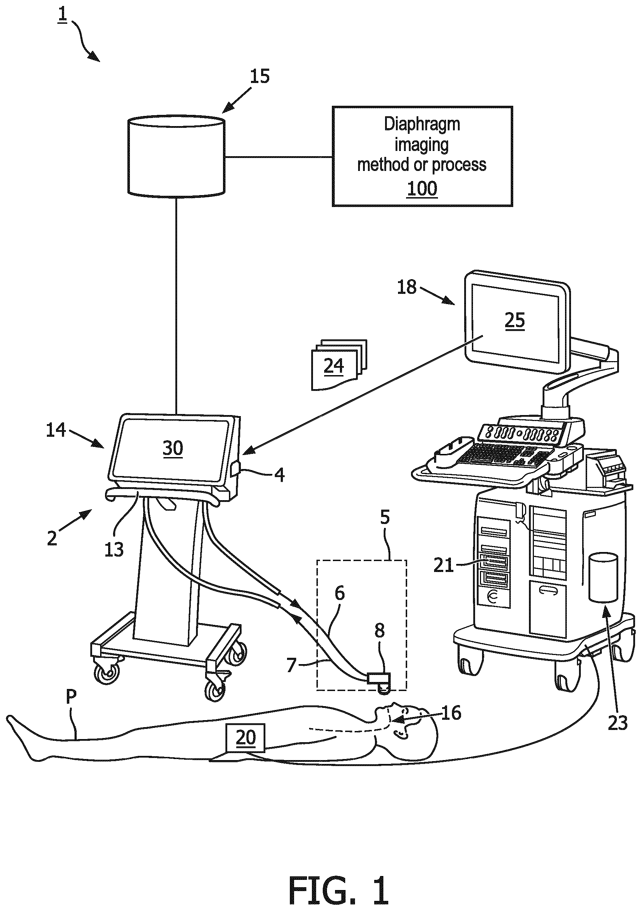

With reference to FIGURE 1, a diaphragm imaging device **1** is shown. A mechanical ventilator **2** is configured to provide ventilation therapy to an associated patient **P** is shown. As shown in FIGURE 1, the mechanical ventilator **2** includes an outlet **4** connectable with a patient breathing circuit **5** to delivery mechanical ventilation to the patient **P.** The patient breathing circuit **5** includes typical components for a mechanical ventilator, such as an inlet line **6, an** optional outlet line **7** (this may be omitted if the ventilator employs a single-limb patient circuit), a connector or port **8** for connecting with an endotracheal tube (ETT) **16,** and one or more breathing sensors (not shown), such as a gas flow meter, a pressure sensor, end-tidal carbon dioxide ($etCO_2$) sensor, and/or so forth. The mechanical ventilator **2** is designed to deliver air, an air-oxygen mixture, or other breathable gas (supply not shown) to the outlet **4** at a programmed pressure and/or flow rate to ventilate the patient via an ETT. The mechanical ventilator **2** also includes at least one electronic processor or controller **13** (e.g., an electronic processor or a microprocessor), a display device **14,** and a non-transitory computer readable medium **15** storing instructions executable by the electronic controller **13.**

FIGURE 1 diagrammatically illustrates the patient **P** intubated with an ETT **16** (the lower portion of which is inside the patient **P** and hence is shown in phantom). The connector or port **8** connects with the ETT **16** to operatively connect the mechanical ventilator **2** to deliver breathable air to the patient **P** via the ETT **16.** The mechanical ventilation provided by the mechanical ventilator **2** via the ETT **16** may be therapeutic for a wide range of conditions, such as various types of pulmonary conditions like emphysema or pneumonia, viral or bacterial infections impacting respiration such as a COVID-19 infection or severe influenza, cardiovascular conditions in which the patient **P** receives breathable gas enriched with oxygen, or so forth.

FIGURE 1 also shows a medical imaging device **18** (also referred to as an image acquisition device, imaging device, and so forth). As primarily described herein, the medical imaging device **18** comprises an ultrasound (US) medical imaging device **18.** The illustrative embodiments employ brightness mode (B-mode) ultrasound imaging to assess the diaphragm thickness metric. However, other types of ultrasound imaging or data are contemplated, such as motion mode (M-mode) data collected as a single ultrasound line over a time interval, or so forth.

In a more particular example, the medical imaging device **18** includes an ultrasound probe **20** that is configured to image the diaphragm of the patient **P.** The US probe **20** is positioned to acquire US imaging data (i.e., US images) **24** of the diaphragm of the patient **P.** For example, the US probe **20** is configured to acquire imaging data of a diaphragm of the patient **P,** and more particularly US imaging data related to a dimension (e.g., a position, a thickness, and so forth) of the diaphragm of a patient **P** during inspiration and expiration while the patient **P** undergoes mechanical ventilation therapy with the mechanical ventilator **2.** In one example, the medical imaging device **18** includes an electronic processor **21** configured to control the ultrasound imaging device **18** to acquire the US images **24,** and also includes a non-transitory computer readable medium **23** storing instructions executable by the electronic processor **21.** The medical imaging device **18** can also include a display device **25.** In another example, the electronic processor **13** of the mechanical ventilator **2** controls the ultrasound imaging device **18** to receive the ultrasound imaging data **24** of the diaphragm of the patient **P** from the US probe **20.** The ultrasound probe **20** allows for continuous and automatic acquisition of the diaphragm thickness data (Tdi) from the acquired ultrasound imaging data **24.**

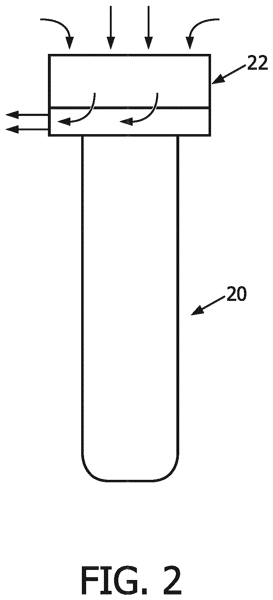

FIGURE 2 shows an example of the ultrasound probe **20.** An orientation of the probe **20** can be adjusted or moved relative to skin of the patient **P** in order to compensate for an inclination angle $\alpha$ of the probe **20** during imaging of the diaphragm of the patient **P.** To do so, the probe **20** includes an actuator **22** configured to move the ultrasound probe **20** relative to the skin of the patient **P.** The actuator **22** can comprise any suitable component, such as a thrust-producing device (i.e., a fan), a gyroscope, and so forth. The actuator **22** is configured to provide thrust (diagrammatically shown in FIGURE 2 with arrows) in order to move (and correctly orientation) the probe **20** relative to the skin of the patient **P** during imaging of the diaphragm.

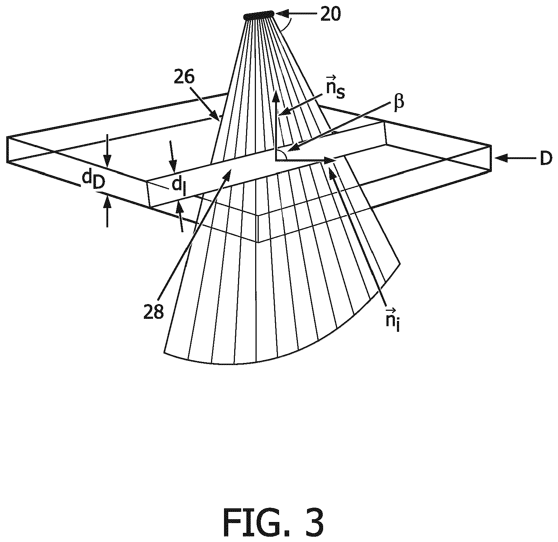

FIGURES 3-5 show example operations of the US probe **20.** As shown in FIGURES 3-5, the ultrasound beam **26** is in the shape of a planar fan emitted from the ultrasound probe **20** towards a surface of a diaphragm **D.** The diaphragm **D** is approximately planar, and has a physical thickness $d_D$ as indicated in FIGURES 3-5. This physical thickness $d_D$ changes over the respiratory cycle, with the physical diaphragm thickness $d_D$ being largest at end-inspiration due to contraction of the diaphragm muscle sheet, and with the physical diaphragm thickness $d_D$ being smallest at end-expiration due to relaxation of the diaphragm. An intersection of the fan of US beams **26** and the diaphragm **D** is denoted in FIGURES 3-5 as diaphragm image or representation **28.** A first normal vector $\vec{n}_i$ is the unit-length normal vector to the plane of the US fan beam **26.** The normal vector $\vec{n}_i$ is thus also the unit-length normal vector to the plane of the image or representation **28** of the diaphragm **D** in the ultrasound image. A second normal vector $\vec{n}_s$ is the unit-length normal vector to the surface of the physical diaphragm **D,** An angle $\beta$ exists between the first and second normal vectors, and the angle $\beta$ is defined as a scalar product of the vectors according to $\beta = \arccos \vec{n}_s \cdot \vec{n}_i$ where "·" denotes the dot product (i.e. scalar product). The physical thickness of the diaphragm **D** is denoted in FIGURES 3-5 as thickness $d_D$. However, an observed thickness $d_I$ of the image **28** of the diaphragm **D** in the US imaging data **24** is broadened by a factor of 1/ sin $|\beta|$ where $|\cdot|$ denotes absolute value. In other words, the observed thickness $d_I$ of the image **28** of the diaphragm **D** in the US imaging data **24** is: $d_I = \dfrac{d_D}{\sin |\beta|}$ . Note that in the limiting case where the US fan beam **26** is oriented at 90° to the plane of the diaphragm **D,** then $\beta$ = 90° and *sin* ($\beta$) = 1 and the imaged diaphragm thickness $d_I$ is equal to the physical diaphragm thickness $d_D$, that is, $d_I = d_D$. However, obtaining this ideal perpendicular orientation is generally difficult or impossible using the external ultrasound probe **20** in an intercostal or subcostal position. Rather, the angle $\beta$ will generally be less than (or greater than) 90° and hence $d_I > d_D$ due to the tilt of the US beam fan **26** away from the perpendicular with the diaphragm **D.**

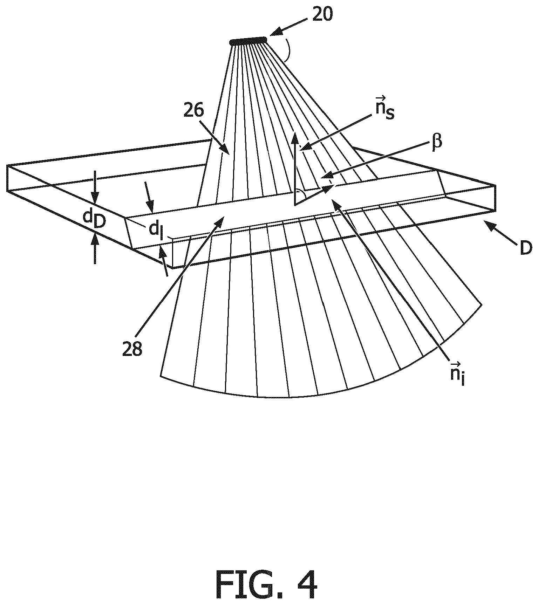

The US probe **20** can be moved by an operator relative to a skin surface of the patient **P** to acquire the US imaging data **24.** If the fan of US beams **26** remains in a single plane, then the angle $\beta$ does not change since the image plane does not change. Thus, the appearance of the thickness $d_I$ of the image **28** of the diaphragm **D** does not change. On the other hand, if the US probe **20** is tilted so that the image plane changes, then the angle $\beta$ changes. This is shown in FIGURE 4, in which the US probe **20** is tilted as opposed to the position of the US probe **20** shown in FIGURE 3. As shown in FIGURE 4, when the US probe **20** is tilted relative to the surface of the diaphragm **D,** the orientation of the first unit normal vector $\vec{n}_i$ of the fan of US beams **26** changes, resulting in a change in the angle $\beta$. The thinnest appearance of the intersection **28** occurs when the angle $\beta$ is 90°, but as previously noted this perpendicular orientation can be difficult or impossible to attain with a physical US probe. Moreover, since the diaphragm **D** is an internal organ, the angle $\beta$ cannot be directly observed or measured.

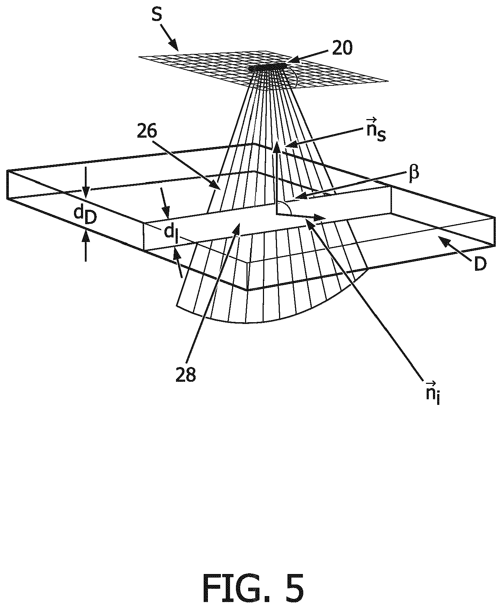

In some examples, one or more external sensors (e.g., a camera - not shown) can be used to determine and angle between the image plan of the fan of US beams **26** and a skin surface **S,** as shown in FIGURE 5. Since the surface S of the skin is in general not parallel to the diaphragm **D,** the angle $\beta$ cannot be directly inferred from an observation of the angle of the ultrasound probe **20** relative to the skin. However, a change in US probe orientation relative to the skin can be correlated with a change in the angle $\beta$ between the plane of the US fan **26** and the plane of the diaphragm **D.** As the ultrasound acquisition frame rate is relatively fast (e.g. typically at least around 10 Hz or higher), by varying the angle of the ultrasound probe **20** with the skin the angle $\beta$ can also be varied.

The apparent (i.e. imaged) diaphragm thickness $d_I = \dfrac{d_D}{\sin |\beta|}$ is measured for several angles of the ultrasound probe **20** respective to the skin (and hence equivalently for several different values of the angle $\beta$) and the smallest value of the apparent (i.e. imaged) diaphragm thickness $d_I$ is taken as being equal to the physical diaphragm thickness $d_D$. This estimated value of $d_D$ may not be exact if the fan beam **26** cannot be positioned to be exactly perpendicular to the plane of the diaphragm **D;** however, since the derivative $\dfrac{d(d_I)}{d\beta}$ becomes small as $\beta$ approaches 90° the estimated value of $d_D$ may have sufficient accuracy. Note that this estimation of $d_D$ is done for different points in the respiratory cycle, typically at least including end-inspiration (where $d_D$ is thickest) and end-expiration (where $d_D$ is thinnest).

If greater accuracy is desired, then the set of datapoints can be extrapolated based on the expectation that the $d_I$ versus US probe angle curve should follow the expected $\frac{1}{\sin\beta}$ shape. For example, denoting the observable angle of the ultrasound probe **20** to the skin as an angle $\beta_{skin}$, several ultrasound images can be acquired at different values of $\beta_{obs}$ to produce a dataset of ($d_I$, $\beta_{obs}$) data pairs. The angle $\beta$ is given as $\beta = \beta_{obs} - \beta_{ref}$ where $\beta_{ref}$ is an (unknown) angle offset between the unobservable angle $\beta$ between the fan beam **26** and the plane of the diaphragm **D** and the observable angle $\beta_{obs}$ between the fan beam **26** and the skin (or other observable US probe angle reference). Then the set of ($d_I$, $\beta_{obs}$) data points are fitted to the equation $d_I = \frac{d_D}{\sin|\beta|}$ with the substitution $\beta = \beta_{obs} - \beta_{ref}$ yielding $d_I = \frac{d_D}{\sin|\beta_{obs}-\beta_{ref}|}$ with the two unknown fitted parameters being $d_D$ and angle $\beta_{ref}$. To accommodate noise in the ($d_I$, $\beta_{obs}$) data points, they can be fitted to a sinusoidal curve which is then matched to $d_I = \frac{d_D}{\sin|\beta_{obs}-\beta_{ref}|}$. Again, this estimation of $d_D$ is done for different points in the respiratory cycle, typically at least including end-inspiration (where $d_D$ is thickest) and end-expiration (where $d_D$ is thinnest). If images are acquired at 10 Hz or faster (i.e., at least 10 images per second) versus a respiratory cycle with a breath rate of typically no faster than about 60 breaths per minute (1 Hz) even for a newborn patient, sufficient data can be collected by sweeping the ultrasound probe **20** over a range of observable angles $\beta_{obs}$ two or three times, e.g. using manual tilting of the US probe **20** with the observable angle $\beta_{obs}$ monitored by an external sensor as described herein.

The non-transitory computer readable medium **15** of the mechanical ventilator **2** and/or the non-transitory computer readable medium **23** of the US imaging device **18** stores instructions executable by the electronic controller **13** (and/or the electronic processor **21**) to perform a diaphragm imaging method or process **100**. Although primarily described in terms of the electronic controller **13**/non-transitory computer readable medium **15** of the mechanical ventilator **2**, the method **100** can similarly be performed by the electronic processor **21**/non-transitory computer readable medium **23** of the US imaging device **18**.

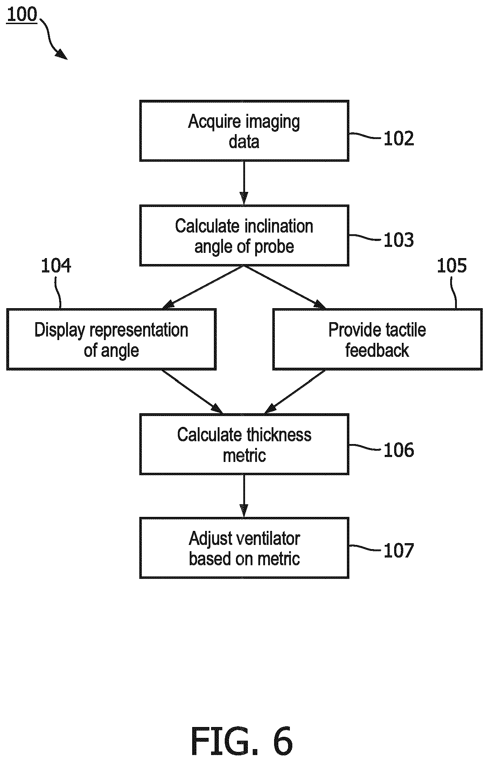

With reference to FIGURE 6, and with continuing reference to FIGURES 1-5, an illustrative embodiment of the diaphragm imaging method **100** is diagrammatically shown as a flowchart. At an operation **102**, the US imaging data **24** of the diaphragm of the patient **P** is acquired with the ultrasound probe **20** and transmitted to the electronic controller **13**. In some embodiments, the US imaging data **24** is acquired while the patient **P** undergoes mechanical ventilation therapy with the mechanical ventilator **2**. The US imaging data **24** includes data related to a geometry (e.g., position, thickness, etc.) of the diaphragm of the patient **P**. To do so, the electronic controller **13** can control the ultrasound probe **20** to acquire the ultrasound imaging data **24** and to receive the ultrasound imaging data **24** of the diaphragm of the patient **P** from the ultrasound probe **20**. These images are not necessarily acquired while the patient **P** is on mechanical ventilation, but instead may be acquired (for example) prior to intubation of the patient.

At an operation **103**, an inclination angle $\beta$ of the ultrasound probe **20** is calculated from the US imaging data **24**. To do so, a position of a surface of the diaphragm of the patient **P** is determined from US imaging data **24**. For example, a clinician can acquire the US imaging data **24** at a plurality of different orientations (i.e., different angles) relative to the skin of the patient **P**. The position of the surface of the diaphragm can be determined from the US imaging data **24** acquired at the plurality of different orientations. From the determined position of the surface of the diaphragm, the inclination angle $\beta$ of the probe **20** can be calculated.

Once the inclination angle $\beta$ of the probe **20** is calculated, a corrective action can be performed. In some embodiments, at an operation **104**, a representation **30** of the calculated inclination angle $\alpha$ can be displayed on the display device **14** of the mechanical ventilator **2** (or on the display device **25** of the medical imaging device **18**). The operator of the probe **20** can then adjust the orientation of the probe **20** relative to the skin of the patient **P** until a desired inclination angle $\beta$ of the probe **20** is achieved. In some examples, a representation **30** of the standard inclination angle $\beta$ can be displayed on the display device **14**, and a representation of the current inclination angle $\beta$ of the probe **20** can also be displayed. The operator can then move the probe **20** until the current inclination angle $\beta$ matches the standard inclination angle $\beta$.

In other embodiments, at an operation **105**, tactile feedback can be provided for an operator of the probe **20**. The tactile feedback can be provided by the actuator **22** of the probe **20** (e.g., the thrust-producing device or gyroscope can vibrate to indicate that the operator should change the orientation of the probe **20**, automatically move the probe **20**, and so forth). In a particular example, a difference between the calculated inclination angle $\beta$ and a standard inclination angle $\beta'$ (i.e., the angle between the normal $\vec{n}_i$ of the image plane and the normal of the skin surface **S**) can be determined, and the tactile feedback can be provided until the calculated inclination angle $\beta$ matches the angle $\beta'$.

At an operation **106**, a diaphragm thickness metric (i.e., a thickness of the diaphragm or a diaphragm thickening fraction) can be calculated based on the US Imaging data **24** and/or the calculated inclination angle $\beta$. The displayed representation **30** can include a representation of the calculated diaphragm thickness metric. In one example, the diaphragm thickness metric includes a diaphragm thickening ratio indicative of a diaphragm thickness during inspiration relative to a diaphragm thickness during expiration. In another example, the diaphragm thickness metric includes a mean diaphragm thickness over multiple respiratory cycles.

At an operation **107,** one or more parameters of the mechanical ventilation therapy delivered to the patient **P** by the mechanical ventilator **2** can be adjusted, for example, based on the calculated diaphragm thickness metric, the inclination angle $\beta$ of the probe **20,** and so forth.

In some embodiments, the actual inclination angle $\beta$ can be estimated, and the operator can either wait for the correct inclination angle $\beta$ of the US beam (i.e., during a manual sweep/attitude variation), or to correct for the artificial thickening (i.e., for a given attitude) caused by the inclination angle $\beta$ of the probe **20.** Since the US imaging data **24** is formed by a divergent set of US beams, the same geometric distortion of the diaphragm thickness appears also for the individual beams. However, the US imaging data **24** is commonly resampled to a Cartesian grid. Thus, the divergence of the beams is compensated, and the diaphragm appears in the image as two straight lines of distance $d/\sin|\beta|$.

The operator continuously changes the inclination angle $\beta$ of the probe **20** in a back-and-forth motion, which can be done at approximately 2 Hz (i.e., as shown in FIGURE 4). The diaphragm thickness is continuously measured within the acquired US images **24** (i.e., as shown in FIGURE 5), and the thinnest estimate is taken (because $d/\sin|\beta|$ has a lower bound of $d$, i.e., the apparent thickness can never be shorter than the true thickness).

The operator continuously changes the inclination angle $\beta$ of the probe **20** back-and-forth, and the diaphragm thickness is continuously measured. Additionally, an inclination angle $\gamma$ of the US probe **20** with respect to the lab coordinate system is measured. This can be done, for instance, using accelerometers or magnetometers (which can measure the angle $\gamma$ between the US probe **20** and the earth's magnetic field) integrated into the US probe **20** or by tracking the probe with external sensors (e. g. optically (i.e., using a camera) or via radiofrequency (RF) tags). The measurement provides pairs of data points (e.g., angle, thickness, etc.). A model of the apparent diaphragm thickness as a function of the actual thickness and the angle is fitted (where the model contains just the artificial thickening by $1/\sin|\beta|$ and the unknown angle between the diaphragm normal and the 0° angle in the lab coordinate system). The model parameter for the actual thickness is provided to the user. The benefit of this approach is that it takes all images from an ultrasound sweep with the probe **20,** resulting in a robust estimate of the inclination angle $\beta$ of the probe **20.**

In an initialization phase of the method **100,** a sweep of the US probe **20** across a broad range of inclination angles $\beta$ can be performed, including tracking of 3D position and orientation of probe **20** (e. g. optically, via RF tags, and so forth) to generate US images. From these images, a position of a local diaphragm surface can be determined. During inhalation and exhalation by the patient **P,** the US images **24** of the diaphragm can be acquired with the probe **20.** The inclination angle $\beta$ of the probe **20** can be estimated with a surface model and the determined local diaphragm surface, and the orientation of the probe **20** can be corrected based on the estimated inclination angle $\beta$.

The tactile feedback operation **105** can be performed in a variety of manners. For example, the US probe **20** is brought into a correct orientation. Then, a gyroscope constituting the actuator **22** is activated, which naturally acts to maintain the probe **20** in its orientation. The gyroscope **22** may be integrated in the US probe **20** or mounted to the US probe **20** as an add-on. For example, to counteract an attitude variation of 5 rad/s with a torque of 0.1 Nm would require a gyroscope **22** with a 5 cm diameter flywheel of 150 g, spinning at 25k RPM. Another approach is to stabilize the US probe **20** by the torque generated from a thrust produced by an axial or centrifugal fan provided as an additional component of the actuator **22.** Again, such a thrust generating device **22** could be integrated in the US probe **20** or mounted to the US probe **20** as an add-on. For example, a thrust of about 1 N (i. e. a torque of about 0.1 Nm when applied at 10 cm from a rotation point) could be generated by a 7.5 x 7.5 cm$^2$ centrifugal fan (e. g. ebm-papst RL 48-19/14) discharging through a 5 mm diameter tube. The active feedback can also be used to ensure the optimal contact area between the US probe **20** and the patient **P.**

In another embodiment, a 3D US probe **20** is employed, or a US probe **20** with two orthogonal fans. The US imaging data **24** is converted into a 3D point cloud, into which two 3D planes are fitted in a robust fashion (using e.g., a RANSAC approach). Finally, the orthogonal plane distance is provided to the operator.

It is beneficial to synchronize the US imaging data **24** with the mechanical ventilator **2** (i. e., thickness measurements are taken automatically in time windows around maximum inspiration and maximum expiration). This synchronization can be done using a common clock. The synchronization between the US probe **20** and the mechanical ventilator **2** allows comparing a measurement from different breaths. If consecutive breaths have similar respiratory muscle activity, points in the respiratory cycle can be selected from multiple breaths in a way that the chosen measurement gives the thickening fraction with the minimum diaphragmatic thickness for every point, this could ensure that the correct inclination angle $\beta$ of the US probe **20** was used. This process can be applied to a subcostal and an intercostal data acquisition scenario.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

**Claims**

1.  A diaphragm imaging device, comprising:
    at least one electronic processor programmed to perform a diaphragm imaging method including:

    receiving ultrasound imaging data of a diaphragm of a patient, the ultrasound imaging data being acquired by an associated ultrasound imaging probe with the probe at a plurality of different observable probe angles ($\beta_{obs}$);
    for each observable probe angle, determining a corresponding apparent thickness ($d_l$) of the diaphragm of the patient from the received ultrasound data acquired at that observable probe angle;
    estimating a thickness ($d_D$) of the diaphragm of the patient based at least on the apparent thicknesses ($d_l$); and
    **characterized in that** the diaphragm imaging method further comprises estimating the thickness ($d_D$) of the diaphragm of the patient as the smallest apparent thickness of the determined apparent thicknesses ($d_l$).

2.  The device of claim 1, wherein the estimating includes:
    fitting data pairs, each comprising one of the determined apparent thicknesses ($d_l$) and its corresponding observable probe angle ($\beta_{obs}$) to an expected relationship between apparent thickness and observable probe angle.

3.  The device of claim 2, wherein an expected relationship between the corresponding apparent thickness ($d_l$) and each observable probe angle ($\beta_{obs}$) is
    $$d_I = \frac{d_D}{\sin|\beta_{obs}-\beta_{ref}|}$$ and the unknown fitted parameters are thickness ($d_D$) of the diaphragm of the patient and a reference angle $\beta_{ref}$.

4.  The device of claim 1, wherein the observable angle ($\beta_{obs}$) is measured during the receiving by:

    determining a position of a surface of the diaphragm of the patient from ultrasound imaging data of the dimension of a diaphragm of a patient; and
    calculating an inclination angle of the associated ultrasound imaging probe (20) from the determined position of the surface of the diaphragm.

5.  The device of claim 4, wherein determining a position of a surface of the diaphragm of the patient from ultrasound imaging data of the dimension of a diaphragm of a patient includes:

    acquiring ultrasound imaging data of the dimension of a diaphragm of a patient based on a plurality of different orientations of the associated ultrasound imaging device relative to skin of the patient;
    determining the position of a surface of the diaphragm from the acquired ultrasound imaging data of the dimension of a diaphragm of a patient.

6.  The device of claim 1, wherein the method further includes performing a corrective action based on the estimated thickness ($d_D$) of the diaphragm, the corrective action comprising:
    displaying, on a display device, a representation of the calculated inclination angle.

7.  The device of claim 1, wherein the method further includes performing a corrective action based on the estimated thickness ($d_D$) of the diaphragm, the corrective action comprising:
    providing, via the associated ultrasound imaging device, tactile feedback for an operator of the associated ultrasound imaging device.

8.  The device of claim 7, wherein the corrective action comprises:

    determining a position of a surface of the diaphragm of the patient from ultrasound imaging data of the dimension of a diaphragm of a patient; and
    determining a standard inclination angle of the associated ultrasound imaging device from the determined position of the surface of the diaphragm at which the associated ultrasound imaging device is able to image the surface of the diaphragm of the patient.

9.  The device of claim 8, wherein the corrective action comprises:

    determining a difference between the calculated inclination angle and the standard inclination angle; and
    providing the tactile feedback until the calculated inclination angle matches the standard inclination angle.

10. The device of claim 1, further including:

    an ultrasound imaging device comprising an ultrasound probe configured to acquire the ultrasound imaging data of the dimension of the diaphragm of the patient;
wherein the ultrasound probe includes an actuator disposed on a portion of the ultrasound probe and configured to move the ultrasound probe relative to skin of the patient.

11. The device of claim 1, wherein calculating an inclination angle of the associated ultrasound imaging device includes:
calculating the inclination angle using one or more sensors.

12. The device of claim 1, wherein the method further includes:

calculating a diaphragm thickness metric based on the received ultrasound imaging data of the diaphragm of the patient; and
displaying, on a display device, a representation of the calculated diaphragm thickness metric.

13. The device of claim 1, further including:
a mechanical ventilator configured to deliver mechanical ventilation therapy to the patient, wherein receiving the ultrasound imaging data of a dimension of a diaphragm of occurs during inspiration and expiration while the patient undergoes mechanical ventilation therapy with the mechanical ventilator; and the method further includes:
controlling an associated mechanical ventilator to adjust one or more parameters of the mechanical ventilation therapy delivered to the patient based on the calculated diaphragm thickness metric .

14. A diaphragm imaging method comprising, with at least one electronic controller:

receiving ultrasound imaging data of a diaphragm of a patient, the ultrasound imaging data being acquired by an associated ultrasound imaging probe with the probe at a plurality of different observable probe angles ($\beta_{obs}$);
for each observable probe angle, determining a corresponding apparent thickness ($d_I$) of the diaphragm of the patient from the received ultrasound data acquired at that observable probe angle;
estimating a thickness ($d_D$) of the diaphragm of the patient based at least on the apparent thicknesses ($d_I$); and
**characterized in that** the diaphragm imaging method further comprises estimating the thickness ($d_D$) of the diaphragm of the patient as the smallest apparent thickness of the determined apparent thicknesses ($d_I$).

**Patentansprüche**

1. Zwerchfellbildgebungsvorrichtung, umfassend:
mindestens einen elektronischen Prozessor, der programmiert ist, um ein Zwerchfellbildgebungsverfahren auszuführen, wobei das Verfahren beinhaltet:

Empfangen von Ultraschallbildgebungsdaten eines Zwerchfells eines Patienten, wobei die Ultraschallbildgebungsdaten von einer zugeordneten Ultraschallbildgebungssonde erfasst werden, wobei sich die Sonde in einer Vielzahl verschiedener beobachtbarer Sondenwinkel ($\beta_{obs}$) befindet;
Bestimmen, für jeden beobachtbaren Sondenwinkel, einer entsprechenden scheinbaren Dicke ($d_I$) des Zwerchfells des Patienten aus den empfangenen Ultraschalldaten, die in diesem beobachtbaren Sondenwinkel erfasst werden;
Schätzen einer Dicke ($d_D$) des Zwerchfells des Patienten mindestens auf den scheinbaren Dicken ($d_I$) basierend, und
**dadurch gekennzeichnet, dass** das Zwerchfellbildgebungsverfahren weiter Schätzen der Dicke ($d_D$) des Zwerchfells des Patienten als die kleinste scheinbare Dicke der bestimmten scheinbaren Dicken ($d_I$) umfasst.

2. Vorrichtung nach Anspruch 1, wobei das Schätzen beinhaltet:
Anpassen von Datenpaaren, die eine der bestimmten scheinbaren Dicken ($d_I$) und ihren entsprechenden beobachtbaren Sondenwinkel ($\beta_{obs}$) umfassen, an eine erwartete Beziehung zwischen scheinbarer Dicke und beobachtbarem Sondenwinkel.

3. Vorrichtung nach Anspruch 2, wobei eine erwartete Beziehung zwischen der entsprechenden scheinbaren Dicke ($d_I$) und jedem beobachtbaren Sondenwinkel ($\beta_{obs}$) $d_I = \dfrac{d_D}{\sin|\beta_{obs} - \beta_{ref}|}$ ist und die unbekannten angepassten Parameter Dicke ($d_D$) des Zwerchfells des Patienten und ein Referenzwinkel $\beta_{ref}$ sind.

4. Vorrichtung nach Anspruch 1, wobei der beobachtbare Winkel ($\beta_{obs}$) während des Empfangs gemessen wird durch:

Bestimmen einer Position einer Oberfläche des Zwerchfells des Patienten aus Ultraschallbildgebungsdaten der Abmessung eines Zwerchfells eines Patienten; und
Berechnen eines Neigungswinkels der zugeordneten Ultraschallbildgebungssonde (20) aus der bestimmten Position der Oberfläche des Zwerchfells.

**5.** Vorrichtung nach Anspruch 4, wobei Bestimmen einer Position einer Oberfläche des Zwerchfells des Patienten aus Ultraschallbildgebungsdaten der Abmessung eines Zwerchfells eines Patienten beinhaltet:

Erfassen von Ultraschallbildgebungsdaten der Abmessung eines Zwerchfells eines Patienten basierend auf einer Vielzahl verschiedener Ausrichtungen der zugeordneten Ultraschallbildgebungsvorrichtung in Bezug zur Haut des Patienten;
Bestimmen der Position einer Oberfläche des Zwerchfells aus den erfassten Ultraschallbildgebungsdaten der Abmessung eines Zwerchfells eines Patienten.

**6.** Vorrichtung nach Anspruch 1, wobei das Verfahren weiter Ausführen einer korrigierenden Aktion basierend auf der geschätzten Dicke ($d_D$) des Zwerchfells beinhaltet, wobei die korrigierende Aktion umfasst: Anzeigen, auf einer Anzeigevorrichtung, einer Darstellung des berechneten Neigungswinkels.

**7.** Vorrichtung nach Anspruch 1, wobei das Verfahren weiter Ausführen einer korrigierenden Aktion basierend auf der geschätzten Dicke ($d_D$) des Zwerchfells beinhaltet, wobei die korrigierende Aktion umfasst: Bereitstellen, über die zugeordnete Ultraschallbildgebungsvorrichtung, einer taktilen Rückmeldung für einen Bediener der zugeordneten Ultraschallbildgebungsvorrichtung.

**8.** Vorrichtung nach Anspruch 7, wobei die korrigierende Aktion umfasst:

Bestimmen einer Position einer Oberfläche des Zwerchfells des Patienten aus Ultraschallbildgebungsdaten der Abmessung eines Zwerchfells eines Patienten; und
Bestimmen eines Standardneigungswinkels der zugeordneten Ultraschallbildgebungsvorrichtung aus der bestimmten Position der Oberfläche des Zwerchfells, an der die zugeordnete Ultraschallbildgebungsvorrichtung imstande ist, die Oberfläche des Zwerchfells des Patienten abzubilden.

**9.** Vorrichtung nach Anspruch 8, wobei die korrigierende Aktion umfasst:

Bestimmen einer Differenz zwischen dem berechneten Neigungswinkel und dem Standardneigungswinkel; und
Bereitstellen der taktilen Rückmeldung, bis der berechnete Neigungswinkel mit dem Standardneigungswinkel übereinstimmt.

**10.** Vorrichtung nach Anspruch 1, die weiter beinhaltet:

eine Ultraschallbildgebungsvorrichtung, die eine Ultraschallsonde umfasst, die konfiguriert ist, um die Ultraschallbildgebungsdaten der Abmessung des Zwerchfells des Patienten zu erfassen;
wobei die Ultraschallsonde eine Betätigungsvorrichtung beinhaltet, der an einem Abschnitt der Ultraschallsonde angeordnet und konfiguriert ist, um die Ultraschallsonde in Bezug zur Haut des Patienten zu bewegen.

**11.** Vorrichtung nach Anspruch 1, wobei Berechnen eines Neigungswinkels der zugeordneten Ultraschallbildgebungsvorrichtung beinhaltet: Berechnen des Neigungswinkels unter Verwendung eines oder mehrerer Sensoren.

**12.** Vorrichtung nach Anspruch 1, wobei das Verfahren weiter beinhaltet:

Berechnen einer Kennzahl der Zwerchfelldicke basierend auf den empfangenen Ultraschallbildgebungsdaten des Zwerchfells des Patienten; und
Anzeigen, auf einer Anzeigevorrichtung, einer Darstellung der berechneten Kennzahl der Zwerchfelldicke.

**13.** Vorrichtung nach Anspruch 1, die weiter beinhaltet: ein mechanisches Beatmungsgerät, das konfiguriert ist, um dem Patienten eine mechanische Beatmungstherapie zu bieten, wobei Empfangen der Ultraschallbildgebungsdaten einer Abmessung eines Zwerchfells beim Einatmen und Ausatmen erfolgt, während sich der Patient einer mechanischen Beatmungstherapie mit dem mechanischen Beatmungsgerät unterzieht; und das Verfahren weiter beinhaltet: Steuern eines zugeordneten mechanischen Beatmungsgeräts, um einen oder mehrere Parameter der mechanischen Beatmungstherapie, die dem Patienten geboten wird, basierend auf der berechneten Kennzahl der Zwerchfelldicke anzupassen.

**14.** Zwerchfellbildgebungsverfahren, umfassend, mit mindestens einer elektronischen Steuereinheit:

Empfangen von Ultraschallbildgebungsdaten eines Zwerchfells eines Patienten, wobei die Ultraschallbildgebungsdaten von einer zugeordneten Ultraschallbildgebungssonde erfasst werden, wobei sich die Sonde in einer Vielzahl verschiedener beobachtbarer Sondenwinkel ($\beta_{obs}$) befindet;
Bestimmen, für jeden beobachtbaren Sondenwinkel, einer entsprechenden scheinbaren Dicke ($d_I$) des Zwerchfells des Patienten aus den empfangenen Ultraschalldaten, die in diesem beobachtbaren Sondenwinkel erfasst werden; Schätzen einer Dicke ($d_D$) des Zwerchfells des Patienten mindestens auf den scheinbaren Dicken ($d_I$) basierend, und
**dadurch gekennzeichnet, dass** das Zwerchfellbildgebungsverfahren weiter Schätzen der Dicke ($d_D$) des Zwerchfells des Patienten als die kleinste scheinbare Dicke der bestimmten scheinbaren Dicken ($d_I$) umfasst.

## Revendications

1. Dispositif d'imagerie de diaphragme, comprenant :
   au moins un processeur électronique programmé pour réaliser un procédé d'imagerie de diaphragme incluant :

   la réception de données d'imagerie ultrasonore d'un diaphragme d'un patient, les données d'imagerie ultrasonore étant acquises par une sonde d'imagerie ultrasonore associée avec la sonde à une pluralité d'angles de sonde observables ($\beta_{obs}$) différents ;
   pour chaque angle de sonde observable, la détermination d'une épaisseur apparente ($d_I$) correspondante du diaphragme du patient à partir des données ultrasonores reçues acquises à cet angle de sonde observable ;
   l'estimation d'une épaisseur ($d_D$) du diaphragme du patient sur la base au moins des épaisseurs apparentes ($d_I$) ; et
   **caractérisé en ce que** le procédé d'imagerie de diaphragme comprend en outre l'estimation de l'épaisseur ($d_D$) du diaphragme du patient comme la plus petite épaisseur apparente des épaisseurs apparentes ($d_I$) déterminées.

2. Dispositif selon la revendication 1, dans lequel l'estimation inclut :
   l'adaptation de paires de données, chacune comprenant une des épaisseurs apparentes ($d_I$) déterminées et son angle de sonde observable ($\beta_{obs}$) correspondant, à une relation attendue entre une épaisseur apparente et un angle de sonde observable.

3. Dispositif selon la revendication 2, dans lequel une relation attendue entre l'épaisseur apparente ($d_I$) correspondante et chaque angle de sonde observable ($\beta_{obs}$) est $d_I = \dfrac{d_D}{\sin|\beta_{obs}-\beta_{ref}|}$ et les paramètres adaptés inconnus sont l'épaisseur ($d_D$) du diaphragme du patient et un angle de référence $\beta_{ref}$.

4. Dispositif selon la revendication 1, dans lequel l'angle observable ($\beta_{obs}$) est mesuré pendant la réception en :

   déterminant une position d'une surface du diaphragme du patient à partir de données d'imagerie ultrasonore de la dimension d'un diaphragme d'un patient ; et
   calculant un angle d'inclinaison de la sonde d'imagerie ultrasonore (20) associée à partir de la position déterminée de la surface du diaphragme.

5. Dispositif selon la revendication 4, dans lequel la détermination d'une position d'une surface du diaphragme du patient à partir de données d'imagerie ultrasonore de la dimension d'un diaphragme d'un patient inclut :

   l'acquisition de données d'imagerie ultrasonore de la dimension d'un diaphragme d'un patient sur la base d'une pluralité d'orientations différentes du dispositif d'imagerie ultrasonore associé par rapport à la peau du patient ;
   la détermination de la position d'une surface du diaphragme à partir des données d'imagerie ultrasonore acquises de la dimension d'un diaphragme d'un patient.

6. Dispositif selon la revendication 1, dans lequel le procédé inclut en outre la réalisation d'une action corrective sur la base de l'épaisseur ($d_D$) estimée du diaphragme, l'action corrective comprenant :
   l'affichage, sur un dispositif d'affichage, d'une représentation de l'angle d'inclinaison calculé.

7. Dispositif selon la revendication 1, dans lequel le procédé inclut en outre la réalisation d'une action corrective sur la base de l'épaisseur ($d_D$) estimée du diaphragme, l'action corrective comprenant :
   la fourniture, via le dispositif d'imagerie ultrasonore associé, d'un retour tactile à un opérateur du dispositif d'imagerie ultrasonore associé.

8. Dispositif selon la revendication 7, dans lequel l'action corrective comprend :

   la détermination d'une position d'une surface du diaphragme du patient à partir de données d'imagerie ultrasonore de la dimension d'un diaphragme d'un patient ; et
   la détermination d'un angle d'inclinaison standard du dispositif d'imagerie ultrasonore associé à partir de la position déterminée de la surface du diaphragme à laquelle le dispositif d'imagerie ultrasonore associé est capable d'imager la surface du diaphragme du patient.

9. Dispositif selon la revendication 8, dans lequel l'action corrective comprend :

la détermination d'une différence entre l'angle d'inclinaison calculé et l'angle d'inclinaison standard ; et

la fourniture du retour tactile jusqu'à ce que l'angle d'inclinaison calculé corresponde à l'angle d'inclinaison standard.

10. Dispositif selon la revendication 1, incluant en outre :

un dispositif d'imagerie ultrasonore comprenant une sonde ultrasonore configurée pour acquérir les données d'imagerie ultrasonore de la dimension du diaphragme du patient ;

dans lequel la sonde ultrasonore inclut un actionneur disposé sur une partie de la sonde ultrasonore et configuré pour déplacer la sonde ultrasonore par rapport à la peau du patient.

11. Dispositif selon la revendication 1, dans lequel le calcul d'un angle d'inclinaison du dispositif d'imagerie ultrasonore associé inclut :

le calcul de l'angle d'inclinaison à l'aide d'un ou plusieurs capteurs.

12. Dispositif selon la revendication 1, dans lequel le procédé inclut en outre :

le calcul d'une mesure d'épaisseur de diaphragme sur la base des données d'imagerie ultrasonore reçues du diaphragme du patient ; et

l'affichage, sur un dispositif d'affichage, d'une représentation de la mesure d'épaisseur de diaphragme calculée.

13. Dispositif selon la revendication 1, incluant en outre : un ventilateur mécanique configuré pour délivrer une thérapie de ventilation mécanique au patient, dans lequel la réception des données d'imagerie ultrasonore d'une dimension d'un diaphragme se produit pendant l'inspiration et l'expiration tandis que le patient subit une thérapie de ventilation mécanique avec le ventilateur mécanique ; et le procédé inclut en outre :

la commande d'un ventilateur mécanique associé pour ajuster un ou plusieurs paramètres de la thérapie de ventilation mécanique délivrée au patient sur la base de la mesure d'épaisseur de diaphragme calculée.

14. Procédé d'imagerie de diaphragme comprenant, avec au moins un dispositif de commande électronique :

la réception de données d'imagerie ultrasonore d'un diaphragme d'un patient, les données d'imagerie ultrasonore étant acquises par une sonde d'imagerie ultrasonore associée avec la sonde à une pluralité d'angles de sonde observables ($\beta_{obs}$) différents ;

pour chaque angle de sonde observable, la détermination d'une épaisseur apparente ($d_l$) correspondante du diaphragme du patient à partir des données ultrasonores reçues acquises à cet angle de sonde observable ;

l'estimation d'une épaisseur ($d_D$) du diaphragme du patient sur la base au moins des épaisseurs apparentes ($d_l$) ; et

**caractérisé en ce que** le procédé d'imagerie de diaphragme comprend en outre l'estimation de l'épaisseur ($d_D$) du diaphragme du patient comme la plus petite épaisseur apparente des épaisseurs apparentes ($d_l$) déterminées.

Diaphragm
imaging
method or process
100

FIG. 1

FIG. 2

FIG. 3

EP 4 590 203 B1

FIG. 4

16

FIG. 5

<u>100</u>

```
                    ┌─────────────────────┐
                    │   Acquire imaging   │
                    │        data         │──── 102
                    └─────────────────────┘
                               │
                               ▼
                    ┌─────────────────────┐
                    │ Calculate inclination│
                    │    angle of probe   │──── 103
                    └─────────────────────┘
        104                 ╱       ╲              105
         │                 ╱         ╲              │
         ▼                ▼           ▼             ▼
┌─────────────────────┐         ┌─────────────────────┐
│Display representation│         │   Provide tactile   │
│       of angle      │         │      feedback       │
└─────────────────────┘         └─────────────────────┘
              ╲                   ╱
               ╲                 ╱
                ▼               ▼
            ┌─────────────────────┐
            │ Calculate thickness │
            │       metric        │──── 106
            └─────────────────────┘
                       │
                       ▼
            ┌─────────────────────┐
            │   Adjust ventilator │
            │   based on metric   │──── 107
            └─────────────────────┘
```

# FIG. 6

## REFERENCES CITED IN THE DESCRIPTION

**Patent documents cited in the description**

- US 63407769

- CN 114680929 A

**Non-patent literature cited in the description**

- **DEMOULE A** ; **HEUNKS L.** Respiratory muscle ultrasonography: methodology, basic and advanced principles and clinical applications in ICU and ED patients - a narrative review.. *Intensive Care Med.*, 14 January 2020, vol. 46 (4), 594-605

- Sonographic evaluation of the diaphragm in critically ill patients. Technique and clinical applications. **MATAMIS DIMITRIOS et al.** INTENSIVE CARE MEDICINE. SPRINGER BERLIN HEIDELBERG, 24 January 2013, vol. 39, 801-810